(19) Europäisches Patentamt
European Patent Office
Office européen des brevets

(11) **EP 3 385 696 B1**

(12) **FASCICULE DE BREVET EUROPEEN**

(45) Date de publication et mention
de la délivrance du brevet:
**23.02.2022 Bulletin 2022/08**

(21) Numéro de dépôt: **18170505.4**

(22) Date de dépôt: **19.12.2013**

(51) Classification Internationale des Brevets (IPC):
*G01N 1/22* *(2006.01)*    *G01M 3/26* *(2006.01)*
*G01N 33/00* *(2006.01)*    *G01N 7/10* *(2006.01)*
*G01N 27/18* *(2006.01)*

(52) Classification Coopérative des Brevets (CPC):
**G01N 7/10; G01N 33/0004;** G01N 27/18

(54) **DISPOSITIF ET PROCEDE DE DETERMINATION DE LA TAILLE D'UN TROU DE FUITE**

VORRICHTUNG UND VERFAHREN ZUR BESTIMMUNG DER GRÖSSE EINER LECKAGE

DEVICE AND METHOD FOR DETERMINING THE SIZE OF A LEAKING HOLE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.12.2012 FR 1262680**

(43) Date de publication de la demande:
**10.10.2018 Bulletin 2018/41**

(62) Numéro(s) de document de la (des) demande(s)
initiale(s) en application de l'article 76 CBE:
**13818740.6 / 2 936 145**

(73) Titulaire: **Aneolia**
**77550 Moissy-Cramayel (FR)**

(72) Inventeurs:
• **GOSSE, Thierry**
**décédé (FR)**
• **LACARRERE, Philippe**
**77380 Combs la Ville (FR)**

• **SCHALLER, Eric**
**91800 Boussy Saint Antoine (FR)**

(74) Mandataire: **IPAZ**
**Parc Les Algorithmes, Bâtiment Platon
CS 70003 Saint-Aubin
91192 Gif-sur-Yvette Cedex (FR)**

(56) Documents cités:
**US-A- 4 776 206        US-A- 5 369 984
US-A1- 2008 092 635**

• **STECKELMACHER W ET AL: "Thermal
conductivity leak detectors suitable for testing
equipment by overpressure or vacuum",
VACUUM, PERGAMON PRESS, GB, vol. 12, no. 3,
1 mai 1962 (1962-05-01), pages 153-159,
XP025620602, ISSN: 0042-207X, DOI:
10.1016/0042-207X(62)90660-7 [extrait le
1962-05-01]**

## Description

### Domaine technique

La présente invention concerne un dispositif de test d'un échantillon. Elle concerne aussi un procédé mis en œuvre par ce dispositif.

Un tel dispositif permet à un utilisateur de tester un échantillon et de mesurer l'intégrité de l'enveloppe de l'échantillon.

### Etat de la technique antérieure

On connaît des systèmes de test d'échantillons, par exemple pour mesurer le taux d'un gaz donné à l'intérieur de l'échantillon ou pour mesurer une fuite ou un problème d'étanchéité de l'échantillon.

Un problème récurrent des solutions de l'état de l'art est qu'elles sont trop chères, trop longues (temps de réponse typique d'une douzaine de secondes pour une mesure par infrarouge de taux de $CO_2$), ou pas assez précises (taille minimum d'un trou de fuite mesurable de 5 $\mu$m avec une surpression relative de 500 mbar, ou avec un balayage d'Hélium dans l'enceinte à mesurer).

US 2008 / 009 26 35 décrit un instrument pour détecter des fuites, utilisant la loi de Poiseuille.

US 5 369 984 décrit un appareil pour déterminer une taille d'un trou de fuite par une méthode par différentiel de pression

Le but de la présente invention est de proposer un dispositif et un procédé de test d'un échantillon ayant au moins un des avantages techniques parmi les suivants :

- faible coût de production par rapport à l'état de l'art,
- grande rapidité d'une mesure par rapport à l'état de l'art, et
- grande résolution de mesure par rapport à l'état de l'art.

### Exposé de l'invention

Cet objectif est atteint avec un dispositif de test d'un échantillon par flux gazeux, comprenant:

- un orifice,
- des moyens pour générer un flux de gaz dans le dispositif le long d'au moins un chemin d'écoulement passant par l'orifice,
- au moins un capteur de pression, chaque capteur de pression étant agencé pour mesurer une pression du flux de gaz le long d'au moins un chemin d'écoulement, et
- un débitmètre massique, agencé pour mesurer un paramètre représentatif du débit massique du flux de gaz le long de chaque chemin d'écoulement, et où
- l'au moins un chemin d'écoulement comprend un chemin d'expiration se terminant par l'orifice,

- les moyens pour générer le flux de gaz sont agencés pour expirer dans l'échantillon un gaz de fuite le long du chemin d'expiration,
- l'au moins un capteur de pression comprend un capteur de pression d'expiration agencé pour mesurer une pression du gaz de fuite le long du chemin d'expiration,
- le débitmètre massique est agencé pour mesurer un paramètre représentatif du débit massique du gaz de fuite le long du chemin d'expiration, et où ledit dispositif est caractérisé en ce qu'il comprend en outre des moyens de calcul agencés pour déterminer la taille d'un trou de fuite, à partir d'une mesure du paramètre représentatif du débit massique le long du chemin d'expiration, le débitmètre massique étant un débitmètre massique à conductibilité thermique.

Le capteur de pression d'expiration est de préférence situé le long du chemin d'expiration entre le débitmètre et l'orifice.

Les moyens de calcul peuvent être agencés pour déterminer la taille du trou de fuite sous la forme d'un calcul qui dépend de manière affine de la racine carrée du paramètre représentatif du débit massique le long du chemin d'expiration.

Les moyens de calcul peuvent être agencés pour déterminer la taille du trou de fuite en outre à partir d'une mesure de pression le long du chemin d'expiration par le capteur de pression d'expiration. Les moyens de calcul peuvent être agencés pour déterminer la taille du trou de fuite sous la forme d'un calcul qui dépend de manière affine de l'inverse de la racine quatrième de la mesure de pression le long du chemin d'expiration. Les moyens de calcul peuvent être agencés pour déterminer la taille du trou de fuite selon la formule $a\dfrac{\sqrt[2]{D_m}}{\sqrt[4]{P_r}}+b$ avec $D_m$ le paramètre représentatif du débit massique, $P_r$ la pression mesurée par le capteur de pression d'expiration, et a et b des coefficients numériques de calibration.

Les moyens de calcul peuvent être agencés pour déclencher une détermination de la taille du trou de fuite pour une valeur de la pression le long du chemin d'expiration mesurée par le capteur de pression d'expiration correspondant à une valeur de référence de pression d'expiration, les moyens de calcul étant agencés pour déterminer la taille du trou de fuite à partir d'une valeur du paramètre représentatif du débit massique le long du chemin d'expiration mesurée simultanément à la mesure de pression mesurant la valeur de pression correspondant à le valeur de référence de pression d'expiration. Les moyens de calcul peuvent être agencés pour déterminer la taille du trou de fuite selon la formule $a^*\sqrt[2]{D_m}+b$ avec $D_m$ le paramètre représentatif du débit massique, et $a^*$ et b des coefficients numériques de calibration.

L'au moins un chemin d'écoulement peut comprendre un chemin de calibration passant par l'orifice, et au sein du dispositif, ledit chemin de calibration peut se rétrécir de manière localisée au niveau d'un trou de mesure, les moyens de calcul étant de préférence agencés pour :

- déterminer la taille du trou de mesure à partir d'une mesure du paramètre représentatif du débit massique le long du chemin de calibration, et
- ajuster des coefficients de calibration pour le calcul d'une taille d'un trou de fuite si la détermination de la taille du trou de mesure ne correspond pas à une taille réelle du trou de mesure mémorisée par les moyens de calcul.

Le dispositif selon l'invention peut comprendre une vanne agencée pour compléter le chemin d'expiration par un chemin de court-circuit passant par l'office et les moyens de génération de flux mais ne passant pas par le débitmètre, ladite vanne étant de préférence agencée pour ajuster le débit passant au total par le chemin d'expiration et le chemin de court-circuit.

L'invention propose aussi un procédé de test d'un échantillon par flux gazeux comprenant:

- une expiration dans l'échantillon d'un gaz de fuite s'écoulant le long d'un chemin d'expiration se terminant par un orifice relié à un échantillon,
- une mesure de pression du gaz de fuite le long du chemin d'expiration,
- une mesure d'un paramètre représentatif du débit massique du gaz de fuite gaz le long du chemin d'expiration, et le procédé étant caractérisé par:
- une détermination de la taille d'un trou de fuite dans l'échantillon, à partir de la mesure du paramètre représentatif du débit massique le long du chemin d'expiration, la mesure d'un paramètre représentatif du débit massique étant une mesure par un débitmètre massique à conductibilité thermique.

La mesure de pression peut être effectuée par un capteur de pression d'expiration situé le long du chemin d'expiration entre le débitmètre et l'échantillon.

La détermination de la taille du trou de fuite peut comprendre un calcul de la taille du trou de fuite qui dépend de manière affine de la racine carrée du paramètre représentatif du débit massique le long du chemin d'expiration.

La détermination de la taille du trou de fuite peut être effectuée en outre à partir de la pression mesurée le long du chemin d'expiration. La détermination de la taille du trou de fuite peut comprendre un calcul de la taille du trou de fuite qui dépend de manière affine de l'inverse de la racine quatrième de la mesure de pression le long du chemin d'expiration. La détermination de la taille du trou de fuite peut comprendre un calcul de la taille du trou de fuite selon la formule $a\dfrac{\sqrt[2]{D_m}}{\sqrt[4]{P_r}}+b$ avec $D_m$ le paramètre représentatif du débit massique, $P_r$ la pression mesurée, et a et b des coefficients numériques de calibration.

La détermination de la taille du trou de fuite peut être déclenchée pour une valeur de la pression le long du chemin d'expiration mesurée correspondant à une valeur de référence de pression, la détermination de la taille du trou de fuite étant effectuée à partir d'une valeur du paramètre représentatif du débit massique le long du chemin d'expiration mesurée simultanément à la mesure de pression mesurant la valeur de pression correspondant à le valeur de référence de pression. La détermination de la taille du trou de fuite peut comprendre un calcul de la taille du trou de fuite selon la formule $a^* \sqrt[2]{D_m} + b$ avec $D_m$ le paramètre représentatif du débit massique, et $a^*$ et $b$ des coefficients numériques de calibration.

L'au moins un chemin d'écoulement peut comprendre un chemin de calibration passant par l'orifice et se rétrécissant de manière localisée au niveau d'un trou de mesure, et le procédé selon l'invention peut comprendre :

- un écoulement d'un gaz de calibration le long du chemin de calibration,
- une mesure de pression du gaz de calibration le long du chemin de calibration,
- une mesure d'un paramètre représentatif du débit massique du gaz de calibration gaz le long du chemin de calibration,
- une détermination de la taille du trou de mesure à partir d'une mesure du paramètre représentatif du débit massique, et
- un ajustement de coefficients numériques pour le calcul d'une taille d'un trou de fuite si la détermination de la taille du trou de mesure ne correspond pas à une taille réelle du trou de mesure mémorisée par des moyens de calcul.

Le procédé selon l'invention peut comprendre un ajustement, par une vanne agencée pour compléter le chemin d'expiration par un chemin de court-circuit passant par l'office et les moyens de génération de flux mais ne passant pas par le débitmètre, du débit passant au total par le chemin d'expiration et le chemin de court-circuit.

## Description des figures et modes de réalisation

D'autres avantages et particularités de l'invention apparaîtront à la lecture de la description détaillée de mises en œuvre et de modes de réalisation nullement limitatifs, et des dessins annexés suivants :

- la figure 1 est une vue schématique de coupe de profil d'un dispositif en train de mettre en œuvre un procédé en dehors du cadre de l'invention, et sur laquelle est illustré un flux de gaz lorsque ce dispositif est dans une position d'analyse de gaz par aspiration ou dans une position de calibration,
- la figure 2 illustre schématiquement le circuit pneumatique du dispositif de la figure 1 dans la position d'analyse de gaz par aspiration ou dans la position de calibration,
- la figure 3 est une vue schématique de coupe de dessous d'une partie du dispositif de la figure 1, et sur laquelle est illustré un flux de gaz lorsque ce dispositif est dans la position d'analyse de gaz par aspiration ou dans la position de calibration,
- la figure 4 illustre schématiquement le circuit pneumatique du dispositif de la figure 1 dans une position de dilution ou dans une autre position de calibration,
- la figure 5 est une vue schématique de coupe de profil du dispositif de la figure 1, en train de mettre en œuvre un procédé selon l'invention et sur laquelle est illustré un flux de gaz lorsque ce dispositif est dans une position de détection de fuite par expiration,
- la figure 6 illustre schématiquement le circuit pneumatique du dispositif de la figure 1 dans la position de détection de fuite par expiration,
- la figure 7 illustre schématiquement le circuit pneumatique du dispositif de la figure 1 dans une position de gonflage rapide par expiration,
- la figure 8 illustre schématiquement le circuit pneumatique du dispositif de la figure 1 dans une position d'éclatement par expiration.

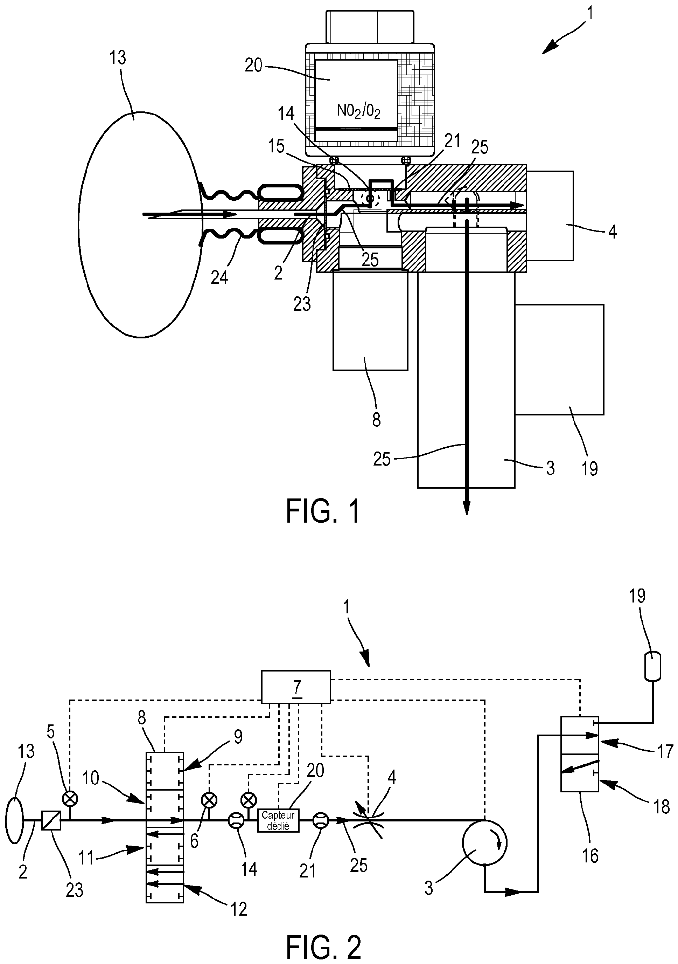

On va tout d'abord décrire, en référence aux figures 1 à 8, un exemple de dispositif 1 se situant en dehors du cadre de l'invention. Le dispositif 1 est un sous-ensemble technique compact pouvant s'installer dans un système portatif ou s'intégrer au sein d'une installation fixe.

Le dispositif 1 est un dispositif de test d'un échantillon par flux gazeux.

Le dispositif 1 comprend un orifice 2. Cet orifice 2 est l'orifice d'entrée du creux d'une aiguille creuse, disposée au centre d'une ventouse 24 étanche agencée pour être plaquée contre un échantillon 13 (tel un sachet de produit alimentaire ou tout contenant présentant au moins une surface souple de dimension compatible pouvant être traversée par une aiguille). La ventouse évite le recours à des septums d'étanchéité pour effectuer un test sans pollution de l'air extérieur au contenant.

Le dispositif 1 comprend en outre des moyens 3 pour générer un flux de gaz 25 (gaz à analyser, gaz de dilution, gaz de fuite, gaz de calibration) dans le dispositif 1 le long d'au moins un chemin d'écoulement passant par l'orifice 2, par un débitmètre massique 4, et par une vanne 8 appelée vanne de sélection.

La vanne 8 est une vanne à plus de deux voies (d'entrée ou de sortie), ayant plusieurs positions possibles. Chaque position de la vanne 8 correspond à une configuration spécifique d'ouverture pour le passage du flux gazeux 25 ou de fermeture pour empêcher un tel passage entre certaines des voies d'entrée et sortie de la vanne 8.

La vanne 8 est de préférence une vanne proportionnelle (de préférence à tiroir).

La vanne 8 est par exemple une vanne réalisée à partir d'un électroaimant de marque Mécalectro, ou encore d'une vanne Parker.

L'orifice 2 et la vanne 8 sont communs à tous les chemins d'écoulement. Le long de cette partie commune des chemins d'écoulement est de préférence situé un élément micro-poreux de filtration 23.

Le filtre 23 est par exemple un filtre en PTFE de chez Millipore ou Sartorius.

Les moyens de génération 3 comprennent une turbine, ou plus généralement un générateur de flux réversible à vitesse contrôlée afin d'être asservie en débit ou en pression, par exemple de marque Papst.

Les moyens de génération 3 sont réversibles, c'est-à-dire qu'ils sont agencés pour générer aussi bien un flux gazeux 25 en aspiration qu'en expiration (i.e. dans un sens d'écoulement opposé à l'aspiration).

Une vanne 16 et l'orifice 2 délimite les deux extrémités de chacun des chemins d'écoulement.

La vanne 16 est une vanne à plus de deux voies (d'entrée ou de sortie), ayant plusieurs positions possibles. Selon la position de la vanne 16, la vanne 16 relie les moyens de génération 3 à l'atmosphère extérieure du dispositif 1 dans une première position 17 ou à une source 19 de gaz de référence dans une deuxième position 18. La vanne 16 est par exemple une vanne de marque Bosch ou bien Univer.

Le dispositif 1 comprend au moins un capteur de pression 5, 6, chaque capteur de pression 5, 6 étant agencé pour mesurer une pression $P_r$ du flux de gaz 25 le long d'au moins un des chemins d'écoulement. Plus exactement, la pression Pr mesurée par chaque capteur 5 ou 6 est une pression relative (respectivement dépression ou surpression) générée par le flux 25 (respectivement aspiré dans le dispositif 1 ou expiré du dispositif 1) par rapport à la pression absolue qui serait mesurée en l'absence de ce flux 25. Chaque capteur 5, 6 est par exemple un capteur piézorésistif de marque Honeywell, Freescale, ou Sensortechnics.

Le débitmètre massique 4 est agencé pour mesurer un paramètre représentatif du débit massique du flux de gaz le long de chaque chemin d'écoulement. Ce paramètre est typiquement une intensité électrique ou une tension électrique, et est de préférence proportionnel au débit massique du flux de gaz 25 ou relié au débit massique du flux de gaz 25 par un calcul programmé et/ou mémorisé au sein de moyens de calcul 7 du dispositif. Tous les éléments sensoriels et d'asservissement 5, 8, 6, 20, 4, 3, 16 sont reliés aux moyens de calcul 7 par une liaison électrique et/ou de transfert de données ou de commande (liaisons représentés en pointillés sur la figure 2). Les moyens de calcul et de commande 7 ne sont représentés schématiquement que sur la figure 2 pour ne pas surcharger les autres figures.

Dans le présent document, le mot « chaque » sert à désigner toute unité (par exemple capteur ou chemin d'écoulement) prise individuellement dans un ensemble. Dans le cas où cet ensemble comprend au moins une unité (i.e. par exemple « au moins un capteur » ou « au moins un chemin d'écoulement »), il existe donc un cas limite où l'ensemble comprend une seule unité (i.e. par exemple un seul capteur ou un seul chemin d'écoulement) et où le mot « chaque » désigne cette seule unité.

Les moyens de calcul 7 ne comprennent que des moyens techniques électroniques et/ou logiciels (de préférence électroniques), et comprennent une unité centrale d'ordinateur, et/ou un processeur, et/ou un circuit analogique ou numérique dédié, et/ou du logiciel.

Le débitmètre massique 4 est un débitmètre massique à conductibilité thermique.

Typiquement, le débitmètre massique 4 comprend un élément chauffant (source de chaleur) et deux sondes de températures. L'élément chauffant se trouve entre les deux sondes de températures de sorte que l'élément chauffant et les deux sondes de températures soient tous les trois alignés le long du sens d'écoulement du flux de gaz 25 au niveau du débitmètre massique. Le débitmètre massique 4, en fonction de la variation de température ou de quantité de chaleur entre les deux sondes de température bordant la source de chaleur, est agencé pour en déterminer le paramètre représentatif du débit massique du flux de gaz 25 passant par le débitmètre 4 (c'est-à-dire une masse de gaz passant par le chemin d'écoulement par unité de temps).

L'avantage d'un débitmètre massique, en particulier à conductibilité thermique, et qu'il a un très rapide temps de réponse. Il va donc permettre d'accéder à un diamètre de trou de fuite 22 ou à une quantification de la présence d'un gaz d'intérêt avec une très grande rapidité de mesure (temps de réponse typique de 3 millisecondes).

L'au moins un chemin d'écoulement comprend :

- un chemin d'aspiration débutant par l'orifice 2 (i.e. le flux de gaz entre dans le chemin d'aspiration par l'orifice 2),
- un chemin d'expiration se terminant par l'orifice 2 (i.e. le flux de gaz sort du chemin d'expiration par l'orifice 2), et
- un chemin de dilution se terminant par l'orifice 2 (i.e. le flux de gaz sort du chemin de dilution par l'orifice 2).

Tous ces chemins d'écoulement sont permis dans le dispositif 1 selon la position de la vanne 8 et le sens du flux 25 généré par les moyens de génération 3. La position de la vanne 8 et le sens du flux 25 généré par les moyens de génération 3 (expiration ou aspiration) à un instant donné détermine l'unique (zéro ou un parmi le chemin d'aspiration, le chemin d'expiration ou le chemin de dilution) chemin d'écoulement à travers lequel s'écoule le flux 25 de gaz à cet instant dans le dispositif 1.

Tous les chemins d'écoulement sont fermés

Pour une première position 9 de la vanne 8, la vanne 8 est fermée et le flux de gaz 25 généré par les moyens 3 ne peut s'écouler le long d'aucun chemin d'écoulement tel que défini précédemment.

Chemin d'aspiration

En référence aux figures 1 à 3, pour une deuxième position 10 de la vanne 8, et pour les moyens de génération 3 aspirant le flux de gaz 25, les moyens 3 pour générer le flux de gaz 25 sont agencés pour aspirer un gaz à analyser en provenance d'un échantillon 13 de sorte que ce gaz à analyser s'écoule dans le dispositif 1 le long du chemin d'aspiration.

Le gaz à analyser comprend par exemple :

- 0 à 100% d'un gaz de mélange comprenant une ou plusieurs molécules (par exemple $N_2$ et $O_2$), chacune de ces molécules ayant avec les autres molécules du gaz de mélange un écart de conductibilité thermique d'au plus 10% (de préférence d'au plus 5%) pour des conditions identiques de température et de pression (typiquement, pour chaque paire de deux molécules du gaz de mélange ayant une conductibilité thermique respectivement $D_i$ et $D_j$ pour des conditions identiques de température (température du flux 25 lors de la mesure de pression $P_r$, typiquement 20°C) et de pression (Pression de mesure $P_r$), on a $\dfrac{D_i - D_j}{D_i} \leq 10\%$ et $\dfrac{D_i - D_j}{Dj} \leq 10\%$, voir de préférence $\dfrac{D_i - D_j}{D_i} \leq 5\%$ et $\dfrac{D_i - D_j}{Dj} \leq 5\%$); ce seuil qui est de manière optimale fixé à 5 ou 10 % peut être aussi supérieur à 10% (20%, 30%, etc...) dans d'autres modes de réalisation, mais plus ce seuil va être élevé, moins bonne va être la résolution du dispositif selon l'invention ; et

- 0 à 100% d'un gaz d'intérêt comprenant uniquement une ou plusieurs molécules (par exemple $NO_2$ et/ou $CO_2$) ayant entre elles un écart de une conductibilité thermique inférieur ou égal à 10% (de préférence inférieur ou égal à 5%) pour des conditions identiques de température et de pression (typiquement, pour chaque paire de deux molécules du gaz d'intérêt ayant une conductibilité thermique respectivement $C_i$ et $C_j$ pour des conditions identiques de température (température du flux 25 lors de la mesure de pression $P_r$, typiquement 20°C) et de pression (Pression de mesure $P_r$), on a $\dfrac{C_i - C_j}{C_i} \leq 10\%$ et $\dfrac{C_i - C_j}{Cj} \leq 10\%$, voir de préférence $\dfrac{C_i - C_j}{C_i} \leq 5\%$ et $\dfrac{C_i - C_j}{Cj} \leq 5\%$). Ce seuil qui est de manière optimale fixé à 5 ou 10 % peut être aussi supérieur à 10% (20%, 30%, etc...) dans d'autres modes de réalisation, mais plus ce seuil va être élevé, moins bonne va être la résolution du dispositif selon l'invention. Chaque molécule du gaz d'intérêt a une conductibilité thermique différente de la conductibilité thermique de chacune des molécules du gaz de mélange d'au moins 20%, de préférence d'au moins 30%, pour des conditions identiques de température et de pression (typiquement, pour chaque molécule du gaz d'intérêt ayant une conductibilité thermique $C_i$ et pour chaque molécule du gaz de mélange ayant une conductibilité thermique $D_i$, pour des conditions identiques de température (température du flux 25 lors de la mesure de pression $P_r$, typiquement 20°C) et de pression (Pression de mesure $P_r$), on a $\dfrac{C_i - D_i}{C_i} \geq 20\%$ et $\dfrac{C_i - D_i}{D_i} \geq 20\%$, voir de préférence $\dfrac{C_i - D_i}{C_i} \geq 30\%$ et $\dfrac{C_i - D_i}{D_i} \geq 30\%$). Cette différence d'au moins 20 ou 30% intervient dans la précision du dispositif 1, plus elle est élevée, plus le gaz d'intérêt est discriminé et réduit le recours à l'amplification électronique ; ce seuil qui est de manière optimale fixé à 20 ou 30 % peut être aussi inférieur à 20% dans d'autres modes de réalisation, mais plus ce seuil va être bas, moins bonne va être la résolution du dispositif selon l'invention ou plus il va falloir une électronique performante pour la discrimination, ou d'autres moyens techniques de réalisation redondants du dispositif décrit dans d'autres échelles de mesure;.

Au sein du dispositif 1, ledit chemin d'aspiration se rétrécit de manière localisée au niveau d'un trou de mesure 14. Le trou de mesure 14 est un trou pratiqué dans une plaque 15. La plaque 15 est typiquement en inox. La plaque 15 est amovible de manière à pouvoir la remplacer typiquement soit en cas d'usure du trou 14 soit pour changer de taille de trou 14 au sein du dispositif 1. Le trou 14 est de dimension connue typiquement de 5 $\mu$m à 150 $\mu$m de diamètre. L'écoulement passe par un deuxième trou 21 de diamètre supérieur (typiquement de l'ordre de 2mm ) au trou de mesure 14. L'épaisseur de cette pastille perforée 15 est un élément d'ajustement de la perte de charge recherchée, et est très inférieure à la taille de l'orifice 14 micro-perforé (typiquement environ 10 fois inférieur)

Ce trou 14 est le passage de plus faible aire d'ouverture (par unité de surface perpendiculaire à la direction du flux 25) pour le flux de gaz 25 dans le dispositif 1 comparé au reste de l'ensemble du chemin d'aspiration, et même de préférence du chemin d'expiration et du chemin de dilution. Typiquement, tous les endroits du chemin d'aspiration (et même de préférence du chemin d'expiration et du chemin de dilution), à l'exception du trou 14 lui-même évidemment, ont une aire d'ouverture (par unité de surface perpendiculaire à la direction du flux 25) au moins 5 fois plus grande que l'aire d'ouverture (par unité de surface perpendiculaire à la direction du flux 25) du trou 14.

Le trou 14 est de forme circulaire.

L'au moins un capteur de pression 5, 6 comprend un premier capteur 6 de pression (dit d'aspiration) agencé pour mesurer une pression $P_r$ (plus exactement une dépression, directement liée à la force d'aspiration de la turbine 3) du gaz à analyser le long du chemin d'aspiration, de préférence mais de manière non limitative comprise entre 20 et 500 mbar ou plus large (comprise entre 4 et 500 mbar ou comprise entre 4 et 1000 mbar ou plus large selon les capacités de la turbine 3).

Le débitmètre massique 4 est agencé pour mesurer le paramètre représentatif du débit massique du gaz à analyser le long du chemin d'aspiration.

Les moyens de calcul 7 sont agencés pour quantifier la présence d'un gaz d'intérêt au sein du gaz à analyser (cette présence quantifiée étant typiquement une proportion en % de gaz d'intérêt dans le gaz à analyser ou en mol par litre de gaz à analyser ou sous la forme d'un volume de gaz d'intérêt par exemple en millilitre), à partir d'une mesure du paramètre représentatif du débit massique du gaz à analyser.

Les moyens de calcul 7 sont agencés pour quantifier la présence du gaz d'intérêt sous la forme d'un calcul d'une proportion ou d'un volume du gaz d'intérêt qui dépend du diamètre du trou 14 de mesure. Autrement dit, si l'on change le diamètre ou la largeur du trou 14 sans l'indiquer (par un programme, une commande, un bouton de réglage, etc...) au dispositif 1, le calcul de proportion ou de volume du gaz d'intérêt par le dispositif 1 devient faux.

Le premier capteur 6 de pression d'aspiration est situé le long du chemin d'aspiration entre l'orifice 2 et le trou de mesure 14, pour une meilleure précision de mesure.

Le débitmètre massique 4 est situé le long du chemin d'aspiration de sorte que le trou de mesure 14 soit situé le long du chemin d'aspiration entre l'orifice 2 et le débitmètre massique 4.

De manière expérimentale, les inventeurs de la présente invention se sont rendu compte qu'une excellente précision de mesure de la taille d'un trou (par exemple de référence 14 ou 22) de rétrécissement au sein d'un écoulement pouvait être atteinte en faisant passer le flux de gaz 25 (typiquement d'air) par ce trou et en mesurant le diamètre de ce trou $\phi_{cal}$ au moyen de la formule suivante :

$$\phi_{cal} = X \sqrt[4]{\frac{D_m^2}{P_r}} + Y = X \frac{\sqrt[2]{D_m}}{\sqrt[4]{P_r}} + Y \qquad \text{(ci après nommée « 1}^{\text{ère}}\text{ formule »)}$$

Avec $D_m$ le paramètre représentatif du débit massique de ce flux de gaz à travers ce trou et $P_r$ la pression de ce flux de gaz, et X et Y des coefficients numériques de calibration.

Pour ce qui est de la mesure de $D_m$, le débitmètre massique 4 est optimisé pour un ou plusieurs types de gaz ayant une valeur par défaut de conductibilité thermique (aussi appelée conductivité thermique). Pour des gaz ayant une conductibilité thermique se démarquant de cette valeur par défaut, un facteur de correction est à appliquer.

Par exemple, dans le cas du débitmètre massique 4 de marque Honeywell de la série AWM, le débit $D_m$ mesuré par ce débitmètre 4 est à multiplier par un facteur 1 (pas de correction) si le flux de gaz est de l'air et/ou $N_2$ et/ou $O_2$ et/ou NO et/ou CO et est à corriger en le multipliant par un facteur de correction $K_{cal}=1,35$ si le flux de gaz est un flux de $CO_2$ et/ou $N_2O$ et/ou $NO_2$, ou un facteur $K_{cal}=0,5$ pour He, $K_{cal}=0,7$ pour $H_2$, $K_{cal}=0,95$ pour Ar, et $K_{cal}=1,1$ pour $CH_4$ et/ou $NH_3$, etc... (se reporter de manière générale à la notice du modèle de débitmètre 4 utilisé).

Considérons comme gaz à analyser un mélange d'$O_2$ et de $CO_2$ provenant de l'échantillon 13 et circulant dans le dispositif 1 le long du chemin d'aspiration ; supposons que l'on sait que ces deux gaz composent le mélange avec chacun une proportion de 0 à 100%, mais que les proportions de ces deux gaz sont inconnues. Considérons le cas où le diamètre réel $\phi_r$ du trou 14 est de 100 $\mu$m.

Si les moyens de calcul 7 calculent, par la 1$^{ère}$ formule précédemment décrite, un diamètre du trou 14 $\phi_{cal}$ de 100 $\mu$m, les moyens de calcul 7 en déduisent soit que la proposition d'$O_2$ dans le mélange est de 100%, soit que la proportion de $CO_2$ dans le mélange est de 0%, selon lequel de ces gaz est considéré comme étant le gaz d'intérêt.

Si les moyens de calcul 7 calculent, par la formule de $\phi_{cal}$ précédemment décrite, un diamètre du trou 14 de 135 $\mu$m, les moyens de calcul 7 en déduisent soit que la proposition d'$O_2$ dans le mélange est de 0%, soit que la proportion de $CO_2$ dans le mélange est de 100%, selon lequel de ces gaz est considéré comme étant le gaz d'intérêt.

De manière générale, si les moyens de calcul 7 calculent, par la formule de $\phi_{cal}$ précédemment décrite, un diamètre du trou 14 de $\phi_{cal}$, les moyens de calcul en déduisent (par une formule ci après nommée « 2$^{ème}$ formule ») que la proposition de $CO_2$ dans le mélange est de $\dfrac{\phi_{cal} - \phi_r}{K_{cal} - 1}$ % ou que la proposition d'$O_2$ dans le mélange est de

$$100 - \dfrac{\phi_{cal} - \phi_r}{K_{cal} - 1} \%$$ selon le gaz d'intérêt considéré, avec Kcal le facteur de correction du gaz d'intérêt comme expliqué précédemment (Kcal=1,35 dans le cas du $CO_2$).

Les moyens de calcul 7 ne sont pas obligés de passer par deux étapes de calcul du diamètre du trou 14 (1$^{ère}$ étape, 1$^{ère}$ formule) puis de déduction de la proportion du gaz d'intérêt (2ème étape, 2ème formule), mais peuvent directement calculer cette proportion en un seul calcul combinant les deux étapes et donc les deux formules.

Les moyens de calcul 7 sont donc agencés pour quantifier la présence du gaz d'intérêt sous la forme d'un calcul d'une proportion ou d'un volume du gaz d'intérêt qui dépend de préférence de manière affine de la racine carrée du paramètre $D_m$ représentatif du débit massique.

Eventuellement, dans le cas moins précis d'un développement limité de la première formule à l'ordre un, les moyens de calcul 7 sont agencés pour quantifier la présence du gaz d'intérêt sous la forme d'un calcul d'une proportion ou d'un volume du gaz d'intérêt qui dépend de manière affine du paramètre représentatif du débit massique.

De manière générale, dans le cas d'un développement limité de la première formule à l'ordre Z (avec Z un entier supérieur ou égal à 1), les moyens de calcul 7 sont agencés pour quantifier la présence du gaz d'intérêt sous la forme d'un polynôme de degré Z du paramètre représentatif du débit massique.

Dans ce contexte, deux variantes, éventuellement combinables au sein d'un même dispositif 1, sont envisageables.

Dans une première variante, les moyens de calcul 7 sont agencés pour quantifier la présence du gaz d'intérêt en outre à partir d'une mesure de pression $P_r$ par le capteur de pression d'aspiration :

- de manière préférentielle, les moyens de calcul 7 sont agencés pour quantifier la présence du gaz d'intérêt sous la forme d'un calcul d'une proportion ou d'un volume du gaz d'intérêt qui dépend de manière affine de l'inverse de la racine quatrième de la mesure de pression par le capteur de pression d'aspiration. La proportion ou le volume du gaz d'intérêt est typiquement calculée selon la formule : $A\dfrac{\sqrt[2]{D_m}}{\sqrt[4]{P_r}} + B$ avec $D_m$ le paramètre représentatif du débit massique mesuré par le débitmètre 4, $P_r$ la pression mesurée par le capteur 6 de pression d'aspiration, et A et B des coefficients numériques de calibration.

- Eventuellement, on peut faire des approximations. Par exemple, les moyens de calcul 7 peuvent être agencés pour quantifier la présence du gaz d'intérêt sous la forme d'un calcul d'une proportion ou d'un volume du gaz d'intérêt qui dépend de manière affine de l'inverse de la mesure de pression par le capteur 6 de pression d'aspiration. La proportion ou le volume du gaz d'intérêt est typiquement calculée selon la formule : $M\dfrac{D_m}{P_r} + N$ avec $D_m$ le paramètre représentatif du débit massique mesuré par le débitmètre 4, $P_r$ la pression mesurée par le capteur 6 de pression d'aspiration, M et N des coefficients numériques de calibration.

Dans une deuxième variante, $P_r$ la pression mesurée par le capteur 6 de pression d'aspiration n'est pas pris en compte dans la formule de calcul de la proportion ou du volume de gaz d'intérêt, mais sert de déclencheur (« trigger ») : les moyens de calcul 7 sont agencés pour déclencher une quantification de la présence du gaz d'intérêt pour une valeur de la pression mesurée $P_r$ par le capteur 6 de pression d'aspiration correspondant à la valeur de référence de pression d'aspiration, les moyens de calcul 7 étant agencés pour quantifier la présence du gaz d'intérêt à partir d'une valeur $D_m$ du paramètre représentatif du débit massique mesurée simultanément à la mesure de pression mesurant la valeur de pression correspondant à la valeur de référence de pression d'aspiration. Les moyens de calcul 7 sont alors agencés pour quantifier la présence du gaz d'intérêt sous la forme d'un calcul d'une proportion ou d'un volume du gaz d'intérêt :

- De préférence selon la formule : $A^* \sqrt[2]{D_m} + B$ avec $D_m$ le paramètre représentatif du débit massique mesuré par le débitmètre 4, et $A^*$ et B des coefficients numériques de calibration.

- Eventuellement selon la formule : $M^* D_m + N$ ou tout autre polynôme de degré Z de $D_m$ comme expliqué précédemment, avec $D_m$ le paramètre représentatif du débit massique mesuré par le débitmètre 4, et $M^*$ et N des coefficients numériques de calibration.

Tous les facteurs de calibration A, B, M, N, $A^*$, $M^*$, a, b, $a^*$ sont mémorisés par les moyens de calcul 7 et sont définis par avance, typiquement en calibrant le dispositif 1 avec des échantillons 13 aux proportions connues de différents gaz ou avec des échantillons 13 munis chacun d'un trou de fuite 22 de dimension connue.

La valeur de chaque facteur de calibration dépend du gaz considéré. Par exemple, on peut présupposer un gaz de mélange d'$O_2$ mélangé avec un gaz d'intérêt de $CO_2$, ou un gaz de mélange d'He mélangé avec un gaz d'intérêt de $CH_4 + NH_3$, etc...

Le dispositif 1 comprend donc une interface agencée pour définir le gaz de mélange et le gaz d'intérêt, et les moyens de calcul 7 sont agencés pour sélectionner les valeurs des facteurs de calibration en fonction des gaz de mélange et d'intérêt définis.

Le chemin d'aspiration passe successivement par l'orifice 2, le filtre 23, le capteur 5 de pression, la vanne 8, le capteur de pression 6, le trou de mesure 14, le capteur 20 de gaz, le trou de passage 21, le débitmètre 4, les moyens de génération 3 et la vanne 16.

Le dispositif 1 comprend en outre au moins un capteur 20 agencé pour quantifier la présence d'un gaz constituée d'une molécule donnée dont la conductibilité thermique ne serait pas discriminée par un autre gaz ou molécule présent(e).

Les moyens de calcul 7 (par exemple dans un cas où le gaz de mélange est de l'$O_2$ et où le gaz d'intérêt est un mélange $CO_2 + NO_2$) sont en outre agencés pour quantifier la présence d'une première molécule d'intérêt (par exemple $CO_2$ dans ce cas) du gaz d'intérêt ayant une certaine conductibilité thermique, le dispositif 1 comprenant pour cela le long du chemin d'aspiration au moins un capteur 20 de gaz (par exemple capteur de $NO_2$ dans ce cas, par exemple de marque City technology) agencé pour quantifier la présence (proportion en % ou en mol.$l^{-1}$ ou volume par exemple) d'au moins une autre molécule d'intérêt (par exemple $NO_2$ dans ce cas) qui a une conductibilité thermique différente au plus de 10% par rapport à la conductibilité thermique de la première molécule d'intérêt pour des conditions de pression et de température identiques, les moyens de calcul 7 étant agencés pour quantifier la présence de la première molécule d'intérêt ($CO_2$) à partir (simple soustraction) d'une quantification de la présence du gaz d'intérêt ($CO_2 + NO_2$) et d'une quantification de la présence des autres molécules d'intérêt ($NO_2$).

Par exemple si on mesure :

Proportion du gaz d'intérêt $CO_2 + NO_2$ = 20% du gaz à analyser
Proportion $NO_2$ = 5% du gaz à analyser

Alors on en déduit :

Proportion du gaz de mélange ($O_2$) = 100 - Proportion $CO_2 + NO_2$ = 80% du gaz à analyser
Proportion $CO_2$ = 15% du gaz à analyser

Le capteur 20 est situé le long du chemin d'aspiration de sorte que le trou de mesure 14 soit situé entre l'orifice 2 et le capteur 20. Le capteur 20 est situé dans une chambre de mesure le long du chemin d'aspiration entre le trou de mesure 14 et un trou de passage 21 plus large que le trou 14 de mesure.

L'au moins un capteur 20 peut aussi être un capteur d'$O_2$, ou autre (par exemple un assemblage capteur d'$O_2$ et capteur de $NO_2$), par exemple si le gaz de mélange comprend un mélange d'$O_2$ et de $N_2$ ceci afin de discriminer ces deux molécules.

Chemin de dilution

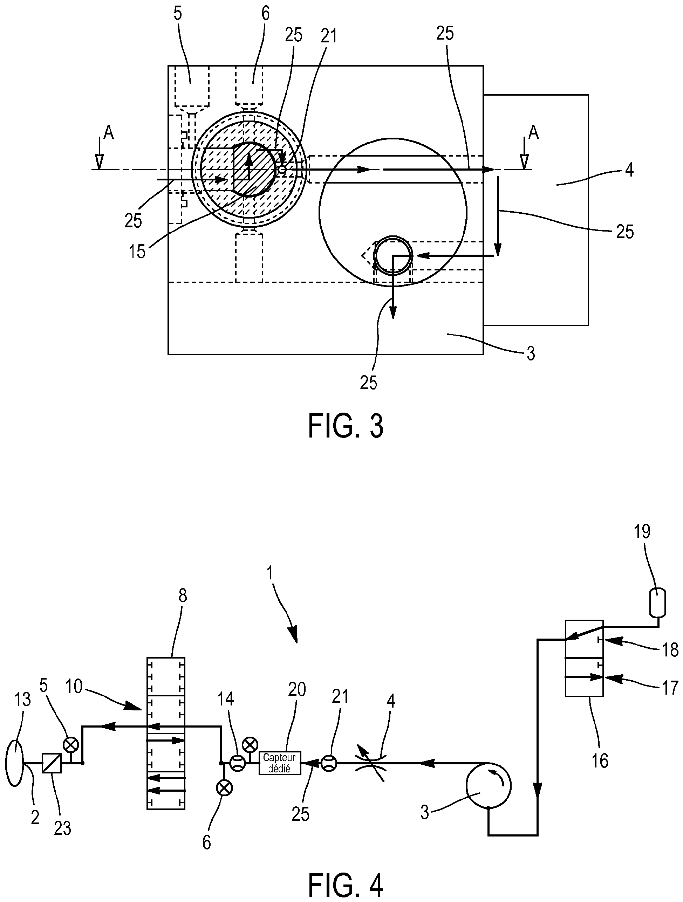

En référence à la figure 4, pour la même position (deuxième position 10) de la vanne 8 que le chemin d'aspiration, et pour les moyens de génération 3 expirant le flux de gaz 25, les moyens 3 pour générer le flux de gaz 25 sont agencés pour expirer un gaz de dilution le long du chemin de dilution.

Le chemin de dilution correspond donc au chemin d'aspiration mais parcouru par le flux de gaz 25 en sens inverse.

Pour le chemin de dilution, la vanne 16 est dans sa deuxième position 18 reliant les moyens 3 à la source 19 de gaz. Le gaz de solution est donc le gaz de référence de la source 19 (qui est typiquement une cartouche de gaz).

Le chemin de dilution sert à augmenter le volume du gaz à analyser de l'échantillon 13.

Chemin de dilution : exemple 1

Imaginons que l'échantillon 13 ne contienne initialement comme gaz initial qu'un mélange de $CO_2$ + $NO_2$ sans $O_2$, mais en trop faible quantité pour pouvoir aspirer ce mélange dans le dispositif 1 en remplissant tout le chemin d'aspiration : il est alors impossible de déterminer les proportions de $CO_2$ et de $NO_2$ en l'état. Par contre, si par le chemin de dilution, on insère dans l'échantillon 13 de l'$O_2$ provenant de la source 19, alors l'échantillon 13 contient un mélange de $CO_2$ + $NO_2$ + $O_2$ dans une quantité suffisante pour faire des mesures. On peut déterminer la proportion de $CO_2$, $NO_2$, et $O_2$ après dilution comme décrit précédemment. On peut alors en déduire la proportion de $CO_2$ et de $NO_2$ avant la dilution.

Par exemple si on mesure :

Proportion $CO_2$+$NO_2$=20% du gaz à analyser après dilution
Proportion $NO_2$= 5% du gaz à analyser après dilution

Alors on en déduit :

Proportion $O_2$=100 - Proportion $CO_2$+$NO_2$=80% du gaz à analyser après dilution
Proportion $CO_2$= 15% du gaz à analyser après dilution

Soit :

Proportion $NO_2$= 25% du gaz initial avant dilution
Proportion $CO_2$= 75% du gaz initial avant dilution

Chemin de dilution : exemple 2

Imaginons que l'échantillon 13 ne contienne initialement comme gaz initial qu'un mélange de $N_2$ et d'$O_2$, mais en trop faible quantité pour pouvoir aspirer ce mélange dans le dispositif 1 en remplissant tout le chemin d'aspiration : il est alors impossible de déterminer les proportions ou volumes d'$O_2$ et de $N_2$ en l'état. Par contre, si par le chemin de dilution, on insère dans l'échantillon 13 du $CO_2$ provenant de la source 19, alors l'échantillon 13 contient un mélange de $CO_2$ + $N_2$ + $O_2$ dans une quantité suffisante pour faire des mesures. On peut déterminer la proportion de $CO_2$, $N_2$, et $O_2$ après dilution comme décrit précédemment en utilisant le volume de gaz injecté et le volume de gaz aspiré. On peut alors en déduire la proportion de $O_2$ et de $N_2$ avant la dilution :

- Stade initial : Le volume de gaz supposé contenu est V1 (typiquement cette problématique se rencontre pour des étuis dont le volume disponible est inférieur à 3ml). Ce volume V1 est inconnu au stade initial. Le volume V1 contient majoritairement du N2 et des traces d'$O_2$ non mesurables du fait du volume de gaz disponible dans le contenant.
- Dilution : on procède à une dilution du volume avec 100% de CO2 par injection d'un volume V2 = 10 ml au moins suffisant pour exciter le capteur d'$O_2$ (repère 20). Le volume V2 est ensuite ré-aspiré.

Les proportions données sont :

Mélange O2+CO2+N2 = 1.34 au lieu de 1.35 (référence N2+O2, air)
La quantité de N2+O2 présent dans le mélange dilué est = (100-1.34x100/1.35)xV2 = 0.00296 x V2=0.0296ml
Le volume de CO2 contenu dans V2 est V2-0.037%xV2 = 9.97ml
La concentration en CO2 dans V2 est devenue 99.704%
La proportion de O2 dans le mélange dilué V2 est donné par le capteur 20 = 0.01%, soit 0.001 ml
La proportion d'$O_2$ dans le volume V1 initial est = 0.001x100/0.0296 = 3.378%
Et on peut en déduire le volume V1 : (100-99.704)*10ml = 2.96 ml

Chemin d'expiration

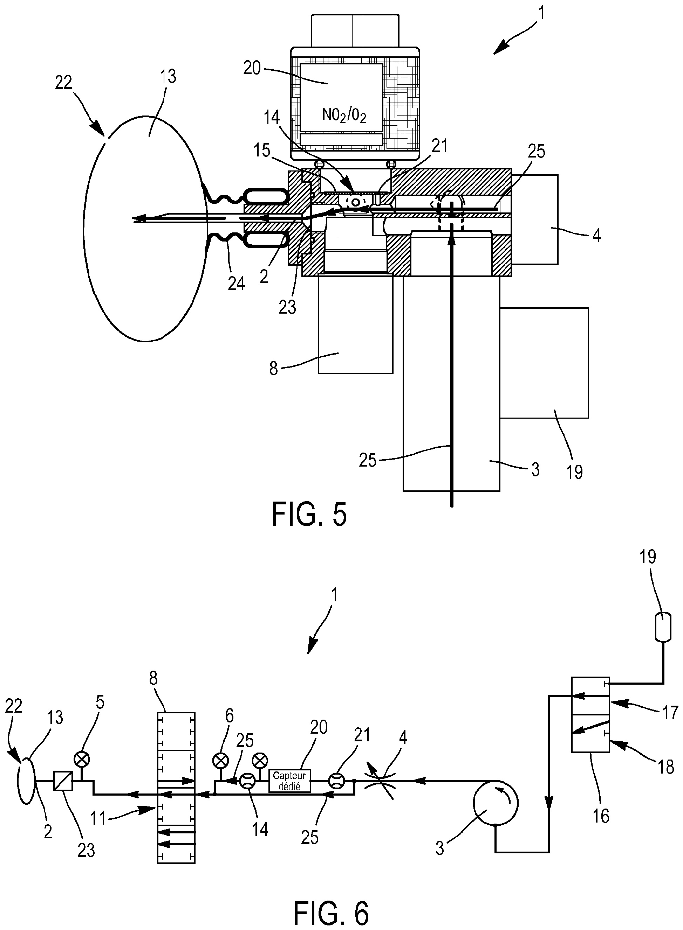

En référence aux figures 5 et 6, pour une troisième position 11 de la vanne 8, et pour les moyens de génération 3 expirant le flux de gaz 25, les moyens 3 pour générer le flux de gaz sont agencés pour expirer un gaz de fuite le long du chemin d'expiration.

Selon la position de la vanne 16, le gaz de fuite (de préférence de l'$O_2$ ou de l'air) provient de l'atmosphère extérieure ou de la source 19.

L'au moins un capteur de pression comprend un capteur 5 de pression d'expiration agencé pour mesurer une pression $P_r$ du gaz de fuite le long du chemin d'expiration, de préférence mais de manière non limitative comprise entre 20 et 500 mbar ou plus large comprise entre 4 et 500 mbar ou comprise entre 4 et 1000 mbar, et dans tous les cas, dans les limites de la perte de charge du circuit pneumatique et de la résistance à la pression des organes constituant l'invention.

Le débitmètre massique 4 est agencé pour mesurer un paramètre représentatif du débit massique du gaz de fuite le long du chemin d'expiration.

Les moyens de calcul 7 sont agencés pour déterminer la taille d'un trou de fuite 22 de l'échantillon 13 (dans lequel est inséré le gaz de fuite expiré par le dispositif 1), à partir d'une mesure du paramètre représentatif du débit massique.

Le capteur 5 de pression d'expiration est situé le long du chemin d'expiration entre le débitmètre 4 et l'orifice 2.

Les moyens de calcul 7 sont agencés pour déterminer la taille du trou de fuite 22 de préférence sous la forme d'un calcul qui dépend de manière affine de la racine carrée du paramètre représentatif du débit massique (cf première formule décrite précédemment).

Eventuellement, dans le cas moins précis d'un développement limité de la première formule à l'ordre un, les moyens de calcul 7 sont agencés pour déterminer la taille du trou de fuite 22 sous la forme d'un calcul qui dépend de manière affine du paramètre représentatif du débit massique.

De manière générale, dans le cas d'un développement limité de la première formule à l'ordre Z (avec Z un entier supérieur ou égal à 1), les moyens de calcul 7 sont agencés pour déterminer la taille du trou de fuite 22 sous la forme d'un polynôme de degré Z du paramètre représentatif du débit massique

Dans ce contexte, deux variantes de l'invention, éventuellement combinables au sein d'un même dispositif 1, sont envisageables.

Dans une première variante, les moyens de calcul 7 sont agencés pour déterminer la taille du trou 22 en outre à partir d'une mesure de pression par le capteur 5 de pression d'expiration, par exemple sous la forme d'un calcul qui dépend de préférence de manière affine de l'inverse de la racine quatrième de la mesure de pression. Typiquement, les moyens de calcul 7 sont agencés pour déterminer la taille du trou 22 selon la formule : $a\dfrac{\sqrt[2]{D_m}}{\sqrt[4]{P_r}}+b$ avec $D_m$ le paramètre représentatif du débit massique mesuré par le débitmètre 4, $P_r$ la pression mesurée par le capteur 5 de pression d'expiration, et a et b des coefficients numériques de calibration.

Selon l'invention, on peut alors mesurer des diamètres de trou de fuite 22 typiquement jusqu'à un minimum de 0,05 $\mu$m.

Dans une deuxième variante, $P_r$ la pression mesurée par le capteur 5 de pression d'expiration n'est pas pris en compte dans la formule de calcul de la taille du trou de fuite 22, mais sert de déclencheur (« trigger ») : les moyens de calcul 7 sont agencés pour déclencher une détermination de la taille du trou 22 pour une valeur de la pression mesurée par le capteur 5 de pression d'expiration correspondant à une valeur de référence de pression d'expiration, les moyens de calcul 7 étant agencés pour déterminer de la taille du trou 22 à partir d'une valeur du paramètre représentatif du débit massique $D_m$ mesurée simultanément à la mesure de pression mesurant la valeur de pression correspondant à le valeur de référence de pression d'expiration. Les moyens de calcul 7 sont par exemple agencés pour déterminer la taille du trou 22 selon la formule : $a^*\sqrt[2]{D_m}+b$ avec $D_m$ le paramètre représentatif du débit massique mesuré par le débitmètre 4, et $a^*$ et b des coefficients numériques de calibration.

Le chemin d'expiration se scinde en deux parties qui se séparent avant le trou de mesure 14 et qui se rejoignent après le trou de mesure 14 :

- une première partie passe par le trou 14 de mesure, (et comprend le capteur 6 et la chambre de mesure comprise entre le trou de mesure 14 et le trou de passage 21)
- une deuxième partie ne passe pas par le trou de mesure 14, pour que le trou de mesure 14 ne limite pas le débit du flux de gaz 25 expiré dans le chemin d'expiration.

Le chemin d'expiration passe donc successivement par la vanne 16, les moyens de génération 3, le débitmètre 4, les deux parties qui se séparent avant le trou de mesure 14 et qui se rejoignent après le trou 14 de mesure, la vanne 8, le capteur 5 de pression, le filtre 23, et l'orifice 2.

Chemin de calibration

L'au moins un chemin d'écoulement comprend un chemin de calibration passant par l'orifice 2, qui correspond au chemin d'aspiration ou de dilution. Ce chemin de calibration se rétrécit de manière localisée au niveau du trou 14 de mesure. Lorsque les moyens 3 de génération génèrent un flux de gaz 25 (gaz de calibration) en aspiration ou en expiration dans ce chemin de calibration sans que l'orifice 2 ne soit connecté à un échantillon fermé 13 (l'orifice 2 débouchant plutôt de préférence à l'air libre), les moyens de calcul 7 sont agencés pour :

1) déterminer la taille du trou 14 de mesure à partir d'une mesure du paramètre représentatif du débit massique $D_m$ par le débitmètre 4, sur le même principe que la détermination de la taille d'un trou de fuite 22 précédemment décrite, et

2) ajuster ses coefficients numériques (typiquement a, b, a*, etc.) pour le calcul d'une taille d'un trou 22 de fuite si sa détermination de la taille $\phi_{cal}$ du trou 14 de mesure ne correspond pas à la taille réelle $\phi_r$ du trou de mesure 14 mémorisée par les moyens 7 de calcul,

3) Et optionnellement ré-itérer les étapes 1) et 2) si dessus jusqu'à ce que la détermination de la taille du trou 14 de mesure corresponde à un pourcentage d'erreur près à la taille réelle du trou de mesure 14 mémorisée par les moyens de calcul.

Chemin de court-circuit

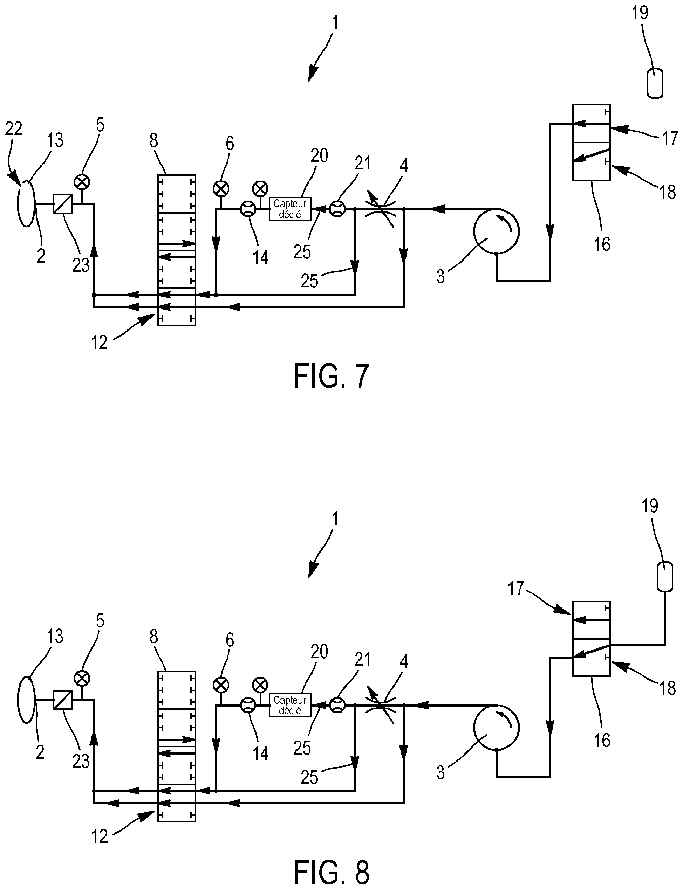

En référence aux figures 7 et 8, la vanne 8 dans sa quatrième position 12 est agencée pour compléter le chemin d'expiration par un chemin de court-circuit passant par l'office 2 et les moyens 3 de génération de flux mais ne passant pas par le débitmètre 4 (ce chemin de court-circuit ne faisant donc pas partie des chemins d'écoulement tel que définis précédemment). La vanne 8 est agencée pour ajuster le débit passant au total par le chemin d'expiration et le chemin de court-circuit. Cela permet des débits $D_m$ plus grand, et donc de mesurer d'autres échelles de diamètre de trou de fuite 22 ou de gonfler rapidement l'échantillon 13 pour tester sa solidité jusqu'à éclatement par phénomène de fatigue ou de stress successifs. Les moyens de calcul 7 sont agencés pour déduire, à partir d'une mesure de débit $D_m$ le long du chemin d'expiration par le débitmètre massique 4, le débit passant au total par le chemin d'expiration et le chemin de court-circuit lorsque la vanne 8 ouvre ce chemin de court-circuit.

Typiquement, les moyens de calcul 7 appliquent une simple multiplication, par un coefficient de calibration, du débit $D_m$ mesuré par le débitmètre massique 4 pour obtenir le débit passant au total par le chemin d'expiration et le chemin de court-circuit lorsque la vanne 8 ouvre ce chemin de court-circuit. Ou bien de préférence, les moyens 7 de calcul modifient la valeur des coefficients de calibration a et a* lors de la détermination de la taille du trou de fuite 22, pour tenir compte que fait que le débit passant au total par le chemin d'expiration et le chemin de court-circuit est plus grand que la mesure, par le débitmètre 4, du paramètre $D_m$ représentatif du débit massique du gaz de fuite gaz le long du chemin d'expiration.

On va maintenant décrire un exemple de procédé dont la séquence peut être modifiée et mis en œuvre par le dispositif 1 des figures 1 à 8. Les types de gaz cités ($O_2$, $CO_2$, $NO_2$ etc..) ne sont qu'illustratifs et peuvent bien évidemment varier.

Dilution

Tout d'abord, avant l'aspiration du gaz à analyser, le procédé comprend une expiration (par les moyens 3) du gaz de dilution ($CO_2$ provenant de la source 19) s'écoulant le long du chemin de dilution jusque dans l'échantillon 13 comprenant un gaz initial (mélange de $CO_2$ et $NO_2$) qui comprend de préférence mais pas nécessairement le gaz d'intérêt.

Analyse de gaz

Après la dilution, le procédé selon l'invention comprend une aspiration (par les moyens 3) du gaz à analyser ($O_2 + CO_2 + NO_2$) en provenance de l'échantillon 13, ledit gaz à analyser aspiré s'écoulant le long du chemin d'aspiration débutant par l'orifice 2 et se rétrécissant de manière localisée au niveau du trou de mesure 14.

Pendant l'aspiration, le procédé comprend simultanément :

- une mesure de pression (plus exactement une dépression de l'aspiration, a priori négative mais considérée en valeur absolue pour les calculs) $P_r$ du gaz à analyser le long du chemin d'aspiration par le capteur 6, de préférence mais de manière non limitative comprise entre -20 et -500 mbar ou plus large comprise entre 4 et 500 mbar ou comprise entre 4 et 1000 mbar ou plus selon la capacité de la turbine 3 ;
- une mesure d'un paramètre représentatif du débit massique du gaz à analyser gaz le long du chemin d'aspiration, par le débitmètre 4.

Le procédé comprend ensuite une quantification, par les moyens de calcul 7, de la présence du gaz d'intérêt ($CO_2$ + $NO_2$) au sein du gaz à analyser ($O_2$ + $CO_2$ + $NO_2$), à partir de cette dernière mesure du paramètre représentatif du débit massique : par exemple proportion $CO_2$+ $NO_2$=20% du gaz à analyser après dilution. La quantification de la présence du gaz d'intérêt comprend un calcul tel que décrit pour la description du dispositif 1.

Le gaz d'intérêt ($CO_2$ + $NO_2$) comprend de 0 à 100% d'une première molécule d'intérêt ($CO_2$) ayant une certaine conductibilité thermique, et de 0 à 100% d'autres molécules d'intérêt ($NO_2$) qui ont une conductibilité thermique différente au plus de 10% par rapport à la conductibilité thermique de la première molécule d'intérêt pour des conditions identiques de température et de pression.

Le procédé comprend en outre (simultanément à la mesure de pression et du paramètre représentatif du débit massique) une quantification de la présence des autres molécules d'intérêt ($NO_2$) au sein du gaz à analyser ($O_2$ + $CO_2$ + $NO_2$) au moyen du capteur 20 : par exemple proportion $NO_2$= 5% du gaz à analyser après dilution.

Le procédé comprend en outre une quantification de la présence de la première molécule d'intérêt ($CO_2$) dans le gaz à analyser ($O_2$ + $CO_2$ + $NO_2$) à partir de la quantification de la présence du gaz d'intérêt ($CO_2$ + $NO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $CO_2$= 15% du gaz à analyser après dilution.

Le procédé comprend en outre une quantification de la présence de la première molécule d'intérêt ($CO_2$) dans le gaz initial ($CO_2$ + $NO_2$) à partir de la quantification de la présence de la première molécule d'intérêt ($CO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $CO_2$= 75% du gaz initial.

Le procédé comprend en outre une quantification de la présence des autres molécules d'intérêt ($NO_2$) dans le gaz initial ($CO_2$ + $NO_2$) à partir de la quantification de la présence de la première molécule d'intérêt ($CO_2$) au sein du gaz à analyser ($O_2$ + $CO_2$ + $NO_2$) et de la quantification de la présence des autres molécules d'intérêt ($NO_2$) au sein du gaz à analyser ($O_2$ + $CO_2$ + $NO_2$) : par exemple proportion $NO_2$= 25% du gaz initial.

Ensuite, vient le test mécanique de l'échantillon 13.

## Calibration de mesure de fuite

Le procédé comprend alors un écoulement (généré par les moyens 3) d'un gaz de calibration (de préférence de l'air extérieur ou le gaz de la source 19) le long du chemin de calibration, et, simultanément à cet écoulement :

1) une mesure de pression $P_r$ du gaz de calibration le long du chemin de calibration par le capteur 5 ou 6, de préférence mais de manière non limitative comprise entre 20 et 500 mbar ou plus large comprise entre 4 et 500 mbar ou comprise entre 4 et 1000 mbar

2) une mesure d'un paramètre représentatif du débit massique du gaz de calibration le long du chemin de calibration, par le débitmètre 4

3) une détermination de la taille du trou de mesure 14 à partir de cette dernière mesure du paramètre représentatif du débit massique, par les moyens de calcul 7, et

4) un ajustement, par les moyens de calcul 7, de coefficients de calibration a, a*, b pour le calcul d'une taille d'un trou de fuite 22 si la détermination $\phi_{cal}$ de la taille du trou de mesure 14 ne correspond pas à une taille réelle $\phi_r$ du trou de mesure 14 mémorisée par les moyens de calcul, et

5) optionnellement une réitération des étapes 1 à 4 précédentes.

## Mesure de fuite

Le procédé selon l'invention comprend ensuite une expiration du gaz de fuite (de préférence de l'air extérieur ou le gaz de la source 19 ou un gaz traceur permettant de localiser la fuite, colorant ou mesurable par d'autres moyens extérieurs) s'écoulant le long du chemin d'expiration se terminant par l'orifice 2.

Pendant l'expiration, le procédé selon l'invention comprend simultanément :

- une mesure de pression $P_r$ du gaz de fuite le long du chemin d'expiration par le capteur 5, de préférence mais de manière non limitative comprise entre 20 et 500 mbar ou plus large comprise entre 4 et 500 mbar ou comprise entre 4 et 1000 mbar, et dans tous les cas, dans les limites de la perte de charge du circuit pneumatique et de la résistance à la pression des organes constituant l'invention.

- une mesure d'un paramètre représentatif du débit massique du gaz de fuite le long du chemin d'expiration, par le débitmètre 4.

Le procédé selon l'invention comprend ensuite une détermination, par les moyens 7 de calcul, de la taille du trou de fuite 22 dans l'échantillon 13, à partir de cette dernière mesure du paramètre représentatif du débit massique.

La détermination de la taille du trou de fuite 22 comprend un calcul tel que décrit pour la description du dispositif 1.

Si le trou de fuite 22 est trop grand, il faut augmenter le débit de gaz de fuite pour rechercher l'atteinte d'une pression de consigne. Le procédé selon l'invention comprend alors un ajustement, par la vanne 8 agencée pour compléter le chemin d'expiration par un chemin de court-circuit passant par l'office et les moyens de génération de flux mais ne passant pas par le débitmètre, du débit passant au total par le chemin d'expiration et le chemin de court-circuit, ladite vanne ouvrant le chemin de court circuit selon une ouverture de taille réglable.

Le procédé selon l'invention comprend alors une détermination, par les moyens 7, et à partir de la mesure de débit le long du chemin d'expiration, du débit passant au total par le chemin d'expiration et le chemin de court-circuit lorsque la vanne ouvre le chemin de court-circuit.

Plus concrètement, les moyens 7 de calcul modifient la valeur des coefficients de calibration a et a* lors de la détermination de la taille du trou de fuite 22, pour tenir compte que fait que le débit passant au total par le chemin d'expiration et le chemin de court-circuit est plus grand que la mesure, par le débitmètre 4, du paramètre $D_m$ représentatif du débit massique du gaz de fuite gaz le long du chemin d'expiration.

Test de Résistance / d'Eclatement

Après avoir déterminé la taille du trou de fuite 22, le débit de gaz est poussé à une forte valeur, éventuellement à débit contrôlé pour tester l'éclatement de l'échantillon 13 dans une dynamique voulue.

L'échantillon 13 peut être soumis à des contraintes mécaniques externes comme une surenveloppe de bridage, la pression atmosphérique, une immersion dans un fluide, etc...

On remarque en outre que les différentes étapes de ce procédé peuvent être inversées, ou faites de manière simultanées ou être optionnelles. Par exemple, l'étape de calibration n'est pas nécessaire avant la mesure de fuite. De même, la mesure de fuite est complètement indépendante de l'analyse de gaz, et la mesure de fuite peut être réalisée avant l'analyse de gaz ou sans l'analyse de gaz. Dans un cas préférentiel, pour gagner du temps, la mesure de fuite peut être réalisée simultanément à la dilution de préférence une fois une pression d'équilibre atteinte, le gaz de dilution expiré jouant aussi le rôle de gaz de fuite expiré.

Bien sûr, l'invention n'est pas limitée aux exemples qui viennent d'être décrits et de nombreux aménagements peuvent être apportés à ces exemples sans sortir du cadre de l'invention.

**Revendications**

1. Dispositif de test d'un échantillon (13) par flux gazeux, comprenant :

   - un orifice (2),
   - des moyens (3) pour générer un flux de gaz (25) dans le dispositif le long d'au moins un chemin d'écoulement passant par l'orifice,
   - au moins un capteur de pression (5, 6), chaque capteur de pression étant agencé pour mesurer une pression du flux de gaz le long d'au moins un chemin d'écoulement, et
   - un débitmètre massique (4), agencé pour mesurer un paramètre représentatif du débit massique du flux de gaz le long de chaque chemin d'écoulement, et où
   - l'au moins un chemin d'écoulement comprend un chemin d'expiration se terminant par l'orifice,
   - les moyens pour générer le flux de gaz sont agencés pour expirer dans l'échantillon un gaz de fuite le long du chemin d'expiration,
   - l'au moins un capteur de pression comprend un capteur de pression d'expiration (5) agencé pour mesurer une pression du gaz de fuite le long du chemin d'expiration,
   - le débitmètre massique est agencé pour mesurer un paramètre représentatif du débit massique du gaz de fuite le long du chemin d'expiration, et où ledit dispositif est **caractérisé en ce qu'**il comprend en outre des moyens de calcul (7) agencés pour déterminer la taille d'un trou de fuite (22), à partir d'une mesure du paramètre représentatif du débit massique le long du chemin d'expiration le débitmètre massique étant un débitmètre massique à conductibilité thermique.

2. Dispositif selon la revendication 1, **caractérisé en ce que** le capteur de pression d'expiration est situé le long du chemin d'expiration entre le débitmètre et l'orifice.

3. Dispositif selon l'une quelconque des revendications précédentes, **caractérisé en ce que** les moyens de calcul sont agencés pour déterminer la taille du trou de fuite sous la forme d'un calcul qui dépend de manière affine de la racine carrée du paramètre représentatif du débit massique le long du chemin d'expiration.

4. Dispositif selon l'une quelconque des revendications précédentes, **caractérisé en ce que** les moyens de calcul sont agencés pour déterminer la taille du trou de fuite en outre à partir d'une mesure de pression le long du chemin d'expiration par le capteur de pression d'expiration.

5. Dispositif selon la revendication 54, **caractérisé en ce que** les moyens de calcul sont agencés pour déterminer la taille du trou de fuite sous la forme d'un calcul qui dépend de manière affine de l'inverse de la racine quatrième de la mesure de pression le long du chemin d'expiration.

6. Dispositif selon la revendication 65, **caractérisé en ce que** les moyens de calcul sont agencés pour déterminer la taille du trou de fuite selon la formule :

$$a \frac{\sqrt[2]{D_m}}{\sqrt[4]{P_r}} + b$$

Avec $D_m$ le paramètre représentatif du débit massique, $P_r$ la pression mesurée par le capteur de pression d'expiration, et a et b des coefficients numériques de calibration.

7. Dispositif selon l'une quelconque des revendications 1 à 43, **caractérisé en ce que** les moyens de calcul sont agencés pour déclencher une détermination de la taille du trou de fuite pour une valeur de la pression le long du chemin d'expiration mesurée par le capteur de pression d'expiration correspondant à une valeur de référence de pression d'expiration, les moyens de calcul étant agencés pour déterminer la taille du trou de fuite à partir d'une valeur du paramètre représentatif du débit massique le long du chemin d'expiration mesurée simultanément à la mesure de pression mesurant la valeur de pression correspondant à le valeur de référence de pression d'expiration.

8. Dispositif selon la revendication 87, **caractérisé en ce que** les moyens de calcul sont agencés pour déterminer la taille du trou de fuite selon la formule :

$$a^* \sqrt[2]{D_m} + b$$

Avec $D_m$ le paramètre représentatif du débit massique, et $a^*$ et b des coefficients numériques de calibration.

9. Dispositif selon l'une quelconque des revendications précédentes, **caractérisé en ce que** l'au moins un chemin d'écoulement comprend un chemin de calibration passant par l'orifice, et **en ce qu'**au sein du dispositif, ledit chemin de calibration se rétrécit de manière localisée au niveau d'un trou de mesure (14), les moyens de calcul étant agencés pour :

- déterminer la taille du trou de mesure à partir d'une mesure du paramètre représentatif du débit massique le long du chemin de calibration, et
- ajuster des coefficients de calibration pour le calcul d'une taille d'un trou de fuite si la détermination de la taille du trou de mesure ne correspond pas à une taille réelle du trou de mesure mémorisée par les moyens de calcul.

10. Dispositif selon l'une quelconque des revendications précédentes, **caractérisé en ce qu'**il comprend une vanne (8) agencée pour compléter le chemin d'expiration par un chemin de court-circuit passant par l'office et les moyens de génération de flux mais ne passant pas par le débitmètre, ladite vanne étant agencée pour ajuster le débit passant au total par le chemin d'expiration et le chemin de court-circuit.

11. Procédé de test d'un échantillon par flux gazeux comprenant:

- une expiration dans l'échantillon d'un gaz de fuite s'écoulant le long d'un chemin d'expiration se terminant par un orifice (2) relié à l'échantillon (13),
- une mesure de pression du gaz de fuite le long du chemin d'expiration,
- une mesure d'un paramètre représentatif du débit massique du gaz de fuite gaz le long du chemin d'expiration,
et le procédé étant **caractérisé par**:
- une détermination de la taille d'un trou de fuite (22) dans l'échantillon, à partir de la mesure du paramètre représentatif du débit massique le long du chemin d'expiration. la mesure d'un paramètre représentatif du débit massique étant une mesure par un débitmètre massique à conductibilité thermique (4).

**12.** Procédé selon la revendication 11, **caractérisé en ce que** la mesure de pression est effectuée par un capteur de pression d'expiration (5) situé le long du chemin d'expiration entre le débitmètre et l'échantillon.

**13.** Procédé selon l'une quelconque des revendications 11 à 12, **caractérisé en ce que** la détermination de la taille du trou de fuite comprend un calcul de la taille du trou de fuite qui dépend de manière affine de la racine carrée du paramètre représentatif du débit massique le long du chemin d'expiration.

**14.** Procédé selon l'une quelconque des revendications 11 à 13, **caractérisé en ce que** la détermination de la taille du trou de fuite est effectuée en outre à partir de la pression mesurée le long du chemin d'expiration.

**15.** Procédé selon la revendication 14, **caractérisé en ce que** la détermination de la taille du trou de fuite comprend un calcul de la taille du trou de fuite qui dépend de manière affine de l'inverse de la racine quatrième de la mesure de pression le long du chemin d'expiration.

**16.** Procédé selon la revendication 15, **caractérisé en ce que** la détermination de la taille du trou de fuite comprend un calcul de la taille du trou de fuite selon la formule :

$$a\,\frac{\sqrt[2]{D_m}}{\sqrt[4]{P_r}} + b$$

Avec $D_m$ le paramètre représentatif du débit massique, $P_r$ la pression mesurée, et a et b des coefficients numériques de calibration.

**17.** Procédé selon l'une quelconque des revendications 11 à 13, **caractérisé en ce que** la détermination de la taille du trou de fuite est déclenchée pour une valeur de la pression le long du chemin d'expiration mesurée correspondant à une valeur de référence de pression, la détermination de la taille du trou de fuite étant effectuée à partir d'une valeur du paramètre représentatif du débit massique le long du chemin d'expiration mesurée simultanément à la mesure de pression mesurant la valeur de pression correspondant à le valeur de référence de pression.

**18.** Procédé selon la revendication 17, **caractérisé en ce que** la détermination de la taille du trou de fuite comprend un calcul de la taille du trou de fuite selon la formule :

$$a^*\,\sqrt[2]{D_m} + b$$

Avec $D_m$ le paramètre représentatif du débit massique, et $a^*$ et b des coefficients numériques de calibration.

**19.** Procédé selon l'une quelconque des revendications 11 à 18, **caractérisé en ce que** l'au moins un chemin d'écoulement comprend un chemin de calibration passant par l'orifice et se rétrécissant de manière localisée au niveau d'un trou de mesure (14), le procédé selon l'invention comprenant :

- un écoulement d'un gaz de calibration le long du chemin de calibration,
- une mesure de pression du gaz de calibration le long du chemin de calibration,
- une mesure d'un paramètre représentatif du débit massique du gaz de calibration gaz le long du chemin de calibration,
- une détermination de la taille du trou de mesure à partir d'une mesure du paramètre représentatif du débit massique, et
- un ajustement de coefficients numériques pour le calcul d'une taille d'un trou de fuite si la détermination de la taille du trou de mesure ne correspond pas à une taille réelle du trou de mesure mémorisée par des moyens de calcul.

20. Procédé selon l'une quelconque des revendications 11 à 19, **caractérisé en ce qu'**il comprend un ajustement, par une vanne (8) agencée pour compléter le chemin d'expiration par un chemin de court-circuit passant par l'office et les moyens de génération de flux mais ne passant pas par le débitmètre, du débit passant au total par le chemin d'expiration et le chemin de court-circuit.

**Patentansprüche**

1. Vorrichtung zum Prüfen eines Probekörpers (13) mittels Gasströmung, die das Folgende aufweist:

   - eine Öffnung (2),
   - Mittel (3) zur Erzeugung eines Gasflusses (25) in der Vorrichtung entlang mindestens eines Strömungswegs durch die Öffnung,
   - mindestens einen Drucksensor (5, 6), wobei jeder Drucksensor so angeordnet ist, dass er den Druck des Gasstroms entlang mindestens eines Strömungswegs misst, und
   - einen Massendurchflussmesser (4), der so angeordnet ist, dass er einen Parameter misst, der für den Massendurchfluss des Gasstroms entlang jedes Durchflusswegs repräsentativ ist, und wobei
   - der mindestens eine Strömungsweg einen Ausströmungsweg umfasst, der in der Öffnung endet,
   - das Mittel zur Erzeugung des Gasflusses so angeordnet ist, dass es ein Leckgas entlang des Ausströmungsweges in den Probekörper abgibt,
   - der mindestens eine Drucksensor einen Ausströmungsdrucksensor (5) umfasst, der so angeordnet ist, dass er einen Druck des Leckgases entlang des Ausströmungswegs misst,
   - der Massendurchflussmesser so angeordnet ist, dass er einen Parameter misst, der für die Massendurchflussrate des Leckgases entlang des Ausströmungswegs repräsentativ ist, und wobei die Vorrichtung **dadurch gekennzeichnet ist, dass** sie

   ferner eine Berechnungseinrichtung (7) umfasst, die so eingerichtet ist, dass sie die Größe einer Leckage-Öffnung (22) aus einer Messung des Parameters bestimmt, der für die Massenstromrate entlang des Ausströmungswegs repräsentativ ist, wobei der Massendurchflussmesser ein Wärmeleitfähigkeits-Massendurchflussmesser ist.

2. Vorrichtung nach Anspruch 1, **dadurch gekennzeichnet, dass** der Ausströmungsdrucksensor entlang des Ausströmungsweges zwischen dem Durchflussmesser und der Öffnung angeordnet ist.

3. Vorrichtung nach einem der vorhergehenden Ansprüche, **dadurch gekennzeichnet, dass** die Berechnungseinrichtung so eingerichtet ist, dass sie die Größe der Leckage-Öffnung mittels einer Berechnung bestimmt, die affin von der Quadratwurzel des Parameters abhängt, der für den Massenstrom entlang des Ausströmungswegs repräsentativ ist.

4. Vorrichtung nach einem der vorhergehenden Ansprüche, **dadurch gekennzeichnet, dass** die Recheneinrichtung so eingerichtet ist, dass sie die Größe der Leckage-Öffnung anhand einer Druckmessung entlang des Ausströmungswegs durch den Ausströmungsdrucksensor bestimmt.

5. Vorrichtung nach Anspruch 4, **dadurch gekennzeichnet, dass** die Berechnungseinrichtung so eingerichtet ist, dass sie die Größe der Leckage-Öffnung in Form einer Berechnung bestimmt, die affin von der Umkehrung der vierten Wurzel der Druckmessung entlang des Ausströmungswegs abhängt.

6. Vorrichtung nach Anspruch 5, **dadurch gekennzeichnet, dass** die Berechnungseinrichtung so beschaffen ist, dass sie die Größe des Leckage-Öffnung nach der folgenden Formel bestimmt:

$$a\frac{\sqrt[2]{D_m}}{\sqrt[4]{P_r}} + b,$$

wobei $D_m$ der für den Massendurchsatz repräsentative Parameter und $P_r$ der vom Ausströmungsdrucksensor ge-

messene Druck ist und a und b numerische Kalibrierkoeffizienten sind.

7. Vorrichtung nach einem der Ansprüche 1 bis 3, **dadurch gekennzeichnet, dass** die Recheneinrichtung ausgebildet ist zum Initiieren einer Bestimmung der Größe der Leckage-Öffnung für einen Wert des Drucks entlang des Ausströmungswegs, der von dem Ausströmungsdrucksensor gemessen wird und einem Ausströmungsdruck-Referenzwert entspricht, wobei die Recheneinrichtung so ausgebildet ist, dass sie die Größe der Leckage-Öffnung aus einem Wert des Parameters bestimmt, der für den Massenstrom entlang des Ausströmungswegs repräsentativ ist und gleichzeitig mit der Druckmessung gemessen wird, die den Druckwert misst, der dem Ausströmungsdruck-Referenzwert entspricht.

8. Vorrichtung nach Anspruch 7, **dadurch gekennzeichnet, dass** die Berechnungsmittel so beschaffen sind, dass sie die Größe der Leckage-Öffnung nach der folgenden Formel bestimmen :

$$a^* \sqrt[2]{D_m} + b \, ,$$

wobei $D_m$ der für den Massendurchsatz repräsentative Parameter und $a^*$ und b numerische Kalibrierkoeffizienten sind.

9. Vorrichtung nach einem der vorhergehenden Ansprüche, **dadurch gekennzeichnet, dass** der mindestens eine Strömungsweg einen Kalibrierungsweg durch die Öffnung umfasst, und dass sich der Kalibrierungsweg innerhalb der Vorrichtung lokal an einer Messöffnung (14) verengt, wobei die Recheneinrichtung so angeordnet ist, dass sie das Folgende durchführt:

- Bestimmen der Größe der Messöffnung anhand einer Messung des für den Massendurchsatz repräsentativen Parameters entlang des Kalibrierungspfads, und
- Anpassung der Kalibrierkoeffizienten zur Berechnung der Größe einer Leckage-Öffnung, wenn die Bestimmung der Größe der Messbohrung nicht mit der tatsächlichen Größe der von den Berechnungsmitteln gespeicherten Messbohrung übereinstimmt.

10. Vorrichtung nach einem der vorhergehenden Ansprüche, **dadurch gekennzeichnet, dass** sie ein Ventil (8) umfasst, das so beschaffen ist, dass es den Ausströmungsweg um einen Kurzschlussweg ergänzt, der durch den Massendurchflussmesser und die Strömungserzeugungseinrichtung, aber nicht durch die Leitung verläuft, wobei das Ventil so beschaffen ist, dass es die insgesamt durch den Ausströmungsweg und den Kurzschlussweg verlaufende Strömungsrate einstellt.

11. Verfahren zum Testen eines Probekörpers mittels eines Gasflusses, umfassend:

- eine Abgabe eines Leckgases in den Probekörper, das entlang eines Ausströmungsweges strömt, der in einer mit dem Probekörper (13) verbundenen Öffnung (2) endet,
- eine Druckmessung des Leckgases entlang des Ausströmungsweges,
- eine Messung eines Parameters, der für den Massendurchsatz des Leckgases entlang des Ausströmungsweges repräsentativ ist, wobei das Verfahren **gekennzeichnet ist durch** ein
- Bestimmen der Größe einer Leckage-Öffnung (22) in dem Probekörper aus der Messung des Parameters, der für die Massenflussrate entlang des Ausströmungswegs repräsentativ ist,
- wobei die Messung eines für den Massendurchsatz repräsentativen Parameters durch einen Wärmeleitfähigkeits-Massendurchflussmesser (4) erfolgt.

12. Verfahren nach Anspruch 11, **dadurch gekennzeichnet, dass** die Druckmessung mittels eines Ausströmungsdrucksensors (5) durchgeführt wird, der entlang des Ausströmungsweges zwischen dem Durchflussmesser und dem Probekörper angeordnet ist.

13. Verfahren nach einem der Ansprüche 11 bis 12, **dadurch gekennzeichnet, dass** die Bestimmung der Größe der Leckage-Öffnung die Berechnung der Größe der Leckage-Öffnung umfasst, die affin von der Quadratwurzel des Parameters abhängt, der für den Massenfluss entlang des Ausströmungswegs repräsentativ ist.

14. Verfahren nach einem der Ansprüche 11 bis 13, **dadurch gekennzeichnet, dass** die Bestimmung der Größe der Leckage-Öffnung ferner anhand des entlang des Ausströmungswegs gemessenen Drucks erfolgt.

15. Verfahren nach Anspruch 1, **dadurch gekennzeichnet, dass** die Bestimmung der Größe der Leckage-Öffnung die Berechnung der Größe der Leckage-Öffnung umfasst, die affin von der Umkehrung der vierten Wurzel der Druckmessung entlang des Ausströmungswegs abhängig ist.

16. Verfahren nach Anspruch 15, **dadurch gekennzeichnet, dass** die Bestimmung der Leckageöffnungs-Größe eine Berechnung der Leckageöffnungs-Größe nach der folgenden Formel umfasst:

$$a\frac{\sqrt[2]{D_m}}{\sqrt[4]{P_r}} + b$$

wobei $D_m$ der für den Massendurchsatz repräsentative Parameter und $P_r$ der gemessene Druck ist und a und b numerische Kalibrierkoeffizienten sind.

17. Verfahren nach einem der Ansprüche 11 bis 13, **dadurch gekennzeichnet, dass** die Bestimmung der Größe der Leckage-Öffnung für einen Wert des gemessenen Drucks entlang des Ausströmungspfads ausgelöst wird, der einem Druckreferenzwert entspricht, wobei die Bestimmung der Größe der Leckage-Öffnung aus einem Wert des Parameters erfolgt, der für den Massenstrom entlang des Ausströmungspfads repräsentativ ist und gleichzeitig mit der Druckmessung gemessen wird, die den Druckwert misst, der dem Druckreferenzwert entspricht.

18. Verfahren nach Anspruch 19, **dadurch gekennzeichnet, dass** die Bestimmung der Leckageöffnungs-Größe eine Berechnung der Leckageöffnungs-Größe nach der folgenden Formel umfasst:

$$a^* \sqrt[2]{D_m} + b \, ,$$

wobei $D_m$ der für den Massendurchsatz repräsentative Parameter ist und $a^*$ und b numerische Kalibrierkoeffizienten sind.

19. Verfahren nach einem der Ansprüche 11 bis 18, **dadurch gekennzeichnet, dass** der mindestens eine Strömungsweg einen Kalibrierungsweg umfasst, der durch die Öffnung verläuft und sich an einer Messöffnung (14) lokal verengt, wobei das erfindungsgemäße Verfahren das Folgende umfasst:

   - ein Abgeben eines Kalibriergases entlang des Kalibrierpfades,
   - eine Druckmessung des Kalibriergases entlang des Kalibrierpfades,
   - eine Messung eines Parameters, der für den Massendurchsatz des Kalibriergases entlang des Kalibrierpfads repräsentativ ist,
   - eine Bestimmung der Größe der Messöffnung anhand einer Messung des für den Massendurchfluss repräsentativen Parameters, und
   - eine Anpassung der numerischen Koeffizienten für die Berechnung der Größe einer Leckage-Öffnung, wenn die Bestimmung der Größe der Messbohrung nicht mit einer tatsächlichen Größe der Messbohrung übereinstimmt, die von Berechnungsmitteln gespeichert wird.

20. Verfahren nach einem der Ansprüche 11 bis 19, **dadurch gekennzeichnet, dass** es die Einstellung der Durchflussmenge, die insgesamt durch den Ausströmungsweg und den Kurzschlussweg fließt, durch ein Ventil (8) umfasst, das so angeordnet ist, dass es den Ausströmungsweg mit einem Kurzschlussweg ergänzt, der durch die Leitung und die Durchflusserzeugungseinrichtung, aber nicht durch den Durchflussmesser verläuft.

**Claims**

1. Device for testing a sample (13) via a gas stream, comprising:

   - an opening (2),

- means (3) for generating a gas stream (25) in the device along at least one flow path passing through the opening,
- at least one pressure sensor (5, 6), each pressure sensor being arranged in order to measure a pressure of the gas stream along at least one flow path, and
- a mass flowmeter (4), arranged in order to measure a parameter representing the mass flow rate of the gas stream along each flow path

and wherein:

- the at least one flow path comprises an exhaust path terminating at the opening,
- the means for generating the gas stream are arranged in order to exhaust in the sample a leakage gas along the exhaust path,
- the at least one pressure sensor comprises an exhaust pressure sensor (5) arranged in order to measure a pressure of the leakage gas along the exhaust path,
- the mass flowmeter is arranged in order to measure a parameter representing the mass flow rate of the leakage gas along the exhaust path,

and wherein the device is **characterized in that** it further comprises calculation means (7) arranged in order to determine the size of a leak hole (22), based on a measurement of the parameter representing the mass flow rate along the exhaust path

the mass flowmeter being a mass flowmeter using thermal conductivity.

2. Device according to claim 1, **characterized in that** exhaust pressure sensor is situated along the exhaust path between the flowmeter and the opening.

3. Device according to any one of the preceding claims, **characterized in that** the calculation means are arranged in order to determine the size of the leak hole in the form of a calculation which depends linearly on the square root of the parameter representing the mass flow rate along the exhaust path.

4. Device according to any one of the preceding claims, **characterized in that** the calculation means are arranged in order to determine the size of the leak hole also based on a measurement of the pressure along the exhaust path by the exhaust pressure sensor.

5. Device according to claim 4, **characterized in that** the calculation means are arranged in order to determine the size of the leak hole in the form of a calculation which depends linearly on the inverse of the fourth root of the measurement of the pressure along the exhaust path.

6. Device according to claim 5, **characterized in that** the calculation means are arranged in order to determine the

$$a\frac{\sqrt[2]{D_m}}{\sqrt[4]{P_r}} + b$$

size of the leak hole according to the formula $\qquad$ with $D_m$ the parameter representing the mass flow rate, $P_r$ the pressure measured by the exhaust pressure sensor, and a and b being numerical calibration coefficients.

7. Device according to any one of claims 1 to 3, **characterized in that** the calculation means are arranged in order to trigger a determination of the size of the leak hole for a value of the pressure along the exhaust path measured by the exhaust pressure sensor corresponding to an exhaust pressure reference value, the calculation means being arranged in order to determine the size of the leak hole based on a value of the parameter representing the mass flow rate along the exhaust path measured simultaneously with the pressure measurement measuring the pressure value corresponding to the exhaust pressure reference value.

8. Device according to claim 7, **characterized in that** the calculation means are arranged in order to determine the size of the leak hole according to the formula $a^* \sqrt[2]{D_m} + b$ with $D_m$ the parameter representing the mass flow rate, and $a^*$ and b being numerical calibration coefficients.

9. Device according to any one of the preceding claims, **characterized in that** the at least one flow path comprises a calibration path passing through the opening, and **in that** within the device, said calibration path narrows locally at a measurement hole (14), the calculation means being arranged in order to:

- determine the size of the measurement hole based on a measurement of the parameter representing the mass flow rate along the calibration path, and
- adjust calibration coefficients for the calculation of a size of a leak hole if the determination of the size of the measurement hole does not correspond to an actual size of the measurement hole stored by the calculation means.

10. Device according to any one of the preceding claims, **characterized in that** it comprises a valve (8) arranged to complete the exhaust path via a short-circuit path passing through the opening and the stream generation means but not passing through the flowmeter, said valve being arranged in order to adjust the total flow rate passing through the exhaust path and the short-circuit path.

11. Process for testing a sample via a gas stream, comprising:

- exhausting in the sample a leakage gas flowing along an exhaust path terminating in an opening (2) linked to the sample (13),
- measuring the pressure of the leakage gas along the exhaust path,
- measuring a parameter representing the mass flow rate of the leakage gas along the exhaust path, and

the process being **characterized in that** it comprises:

- determining the size of a leak hole (22) in the sample, based on the measurement of the parameter representing the mass flow rate along the exhaust path

the measurement of a parameter representing the mass flow rate being a measurement by a mass flowmeter (14) using thermal conductivity.

12. Process according to claim 11, **characterized in that** the pressure measurement is carried out by an exhaust pressure sensor (5) situated along the exhaust path between the flowmeter and the sample.

13. Process according to any one of claims 11 to 12, **characterized in that** the determination of the size of the leak hole comprises a calculation of the size of the leak hole which depends linearly on the square root of the parameter representing the mass flow rate along the exhaust path.

14. Process according to any one of claims 11 to 13, **characterized in that** the determination of the size of the leak hole is carried out also based on the pressure measured along the exhaust path.

15. Process according to claim 14, **characterized in that** the determination of the size of the leak hole comprises a calculation of the size of the leak hole which depends linearly on the inverse of the fourth root of the measurement of the pressure along the exhaust path.

16. Process according to claim 15, **characterized in that** the determination of the size of the leak hole comprises a calculation of the size of the leak hole according to the formula $a\dfrac{\sqrt[2]{D_m}}{\sqrt[4]{P_r}} + b$ with $D_m$ the parameter representing the mass flow rate, $P_r$ the pressure measured, and a and b being numerical calibration coefficients.

17. Process according to any one of claims 11 to 13, **characterized in that** the determination of the size of the leak hole is triggered in the case of a measured value of the pressure along the exhaust path corresponding to a pressure reference value, the determination of the size of the leak hole being carried out based on a value of the parameter representing the mass flow rate along the exhaust path measured simultaneously with the pressure measurement measuring the pressure value corresponding to the pressure reference value.

18. Process according to claim 17, **characterized in that** the determination of the size of the leak hole comprises a calculation of the size of the leak hole according to the formula $a^* \sqrt[2]{D_m} + b$ with $D_m$ the parameter representing the mass flow rate, and $a^*$ and $b$ being numerical calibration coefficients.

19. Process according to any one of claims 11 to 18, **characterized in that** the at least one flow path comprises a calibration path passing through the opening and narrowing locally at a measurement hole, the process according to the invention comprising:

- a calibration gas flowing along the calibration path,
- a pressure measurement of the calibration gas along the calibration path,
- a measurement of a parameter representing the mass flow rate of the calibration gas along the calibration path,
- a determination of the size of the measurement hole based on a measurement of the parameter representing the mass flow rate, and
- an adjustment of numerical coefficients for the calculation of a size of a leak hole if the determination of the size of the measurement hole does not correspond to an actual size of the measurement hole stored by calculation means.

20. Process according to any one of claims 11 to 19, **characterized in that** it comprises an adjustment, by a valve (8) arranged in order to complete the exhaust path via a short-circuit path passing through the opening and the stream generation means but not passing through the flowmeter, of the total flow rate passing through the exhaust path and the short-circuit path.

FIG. 1

FIG. 2

# FIG. 3

Capteur dédié

# FIG. 4

FIG. 5

FIG. 6

FIG. 7

FIG. 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20080092635 A

- US 5369984 A